# EUROPEAN PATENT SPECIFICATION

(11) **EP 3 915 637 B1**
(45) Date of publication and mention of the grant of the patent: **13.11.2024**
(21) Application number: 19912031.2
(22) Date of filing: 29.11.2019
(51) Int. Cl.: A61K 33/00, A61K 9/51, A61K 9/14, A61K 47/02, A61P 15/08

(54) **SILICON FINE PARTICLES FOR USE IN TREATING VARICOCELE AS A REPRODUCTIVE DISORDER OF MALE INFERTILITY**
FEINE SILIZIUMPARTIKEL ZUR VERWENDUNG BEI DER BEHANDLUNG VON VARIKOZELE ALS FORTPFLANZUNGSSTÖRUNG MÄNNLICHER UNFRUCHTBARKEIT
PARTICULES FINES DE SILICIUM DESTINÉES À ÊTRE UTILISÉES DANS LE TRAITEMENT DE LA VARICOCÈLE EN TANT QUE TROUBLE DE LA REPRODUCTION LIÉ À L'INFERTILITÉ MASCULINE

(30) Priority: 24.01.2019 JP 2019010412
(43) Date of publication of application: 01.12.2021
(73) Proprietor: Osaka University, Suita-shi, Osaka 565-0871 (JP)
(72) Inventor: IMAMURA Ryoichi, Suita-shi, Osaka 565-0871 (JP); KOBAYASHI Hikaru, Suita-shi, Osaka 565-0871 (JP); KOBAYASHI Yuki, Suita-shi, Osaka 565-0871 (JP); FUKUHARA Shinichiro, Suita-shi, Osaka 565-0871 (JP); INAGAKI Yusuke, Suita-shi, Osaka 565-0871 (JP); NONOMURA Norio, Suita-shi, Osaka 565-0871 (JP)
(74) Representative: Schön, Christoph
(86) International application number: PCT/JP2019/046828
(87) International publication number: WO 2020/152985

(56) References cited:
- EP-A1- 3 409 282
- EP-A1- 3 659 610
- WO-A1-2017/130709
- WO-A1-2018/037752
- US-B2- 8 980 847
- KUMIKO NAKATA ET AL: "Stimulation of human damaged sperm motility with hydrogen molecule", MEDICAL GAS RESEARCH, BIOMED CENTRAL LTD, LONDON, UK, vol. 5, no. 1, 10 January 2015 (2015-01-10), pages 2, XP021210549, ISSN: 2045-9912, DOI: 10.1186/S13618-014-0023-X
- HE XIN ET AL: "Hydrogen-rich water exerting a protective effect on ovarian reserve function in a mouse model of immune premature ovarian failure induced by zona pellucida 3", CHINESE MEDICAL JOURNAL V, vol. 129, no. 19, 5 October 2016 (2016-10-05), pages 2331 - 2337, XP055887705, Retrieved from the Internet <URL:https://www.ncbi.nlm.nih.gov/pmc/articles/PMC5040020/pdf/CMJ-129-2331.pdf> DOI: 10.4103/0366-6999.190668
- BEGUM RAHIMA ET AL: "Molecular hydrogen may enhance the production of testosterone hormone in male infertility through hormone signal modulation and redox balance", MEDICAL HYPOTHESES, EDEN PRESS, PENRITH, US, vol. 121, 6 September 2018 (2018-09-06), pages 6 - 9, XP085532146, ISSN: 0306-9877, DOI: 10.1016/J.MEHY.2018.09.001
- KIN, SEITETSU ET AL.: "PP56-01 Study on the effect of hydrogen water under oxidative stress environment using rat cryptorchidism model", ABSTRACT OF THE 105TH ANNUAL MEETING OF THE JAPANESE UROLOGICAL ASSOCIATION; APRIL 21-24, 2017, vol. 105, 30 November 2016 (2016-11-30) - 24 April 2017 (2017-04-24), JP, pages 1251, XP009529266
- NODA, YOSHIHIRO ET AL.: "O-56 Decreasing of mouse sperm motility function and suppressive effect of molecular hydrogen by active oxygen disorder", LECTURE ABSTRACTS OF 63RD ANNUAL MEETING OF JAPANESE ASSOCIATION FOR LABORATORY ANIMAL SCIENCE; MAY 18-20, 2016, vol. 63, 22 April 2016 (2016-04-22) - 20 May 2016 (2016-05-20), JP, pages 138, XP009529267
- BEGUM, R. ET AL.: "Molecular hydrogen may enhance the production of testosterone hormone in male infertility through hormone signal modulation and redox balance", MEDICAL HYPOTHESES, vol. 121, 2018, pages 6 - 9, XP085532146, ISSN: 0306-9877, DOI: 10.1016/j.mehy.2018.09.001

## Description

### TECHNICAL FIELD

The present invention relates to a drug for use in treating varicocele as a reproductive disorder of male infertility comprising silicon fine particles as defined in the claims.

### BACKGROUND ART

Efforts for prevention or treatment of reproductive disorder (also referred to as "reproductive dysfunction") have been actively made in various countries, but as far as the present inventors are aware, no causal therapy for reproductive disorder has been found yet. Consideration of varicocele as an example of reproductive disorder suggests that there is only surgical therapy as a radical treatment method for varicocele, which is actually applied to humans as of the time when the present application is filed.

Some of the present inventors have so far advanced researches for preventing hydroxyl radicals from being present in the body. Among superoxide anion radicals as active oxygen, hydroxyl radicals, hydrogen peroxide, and singlet oxygen, the hydroxyl radicals have the strongest oxidizing power without having a physiological function.

Hydrogen is known as an example of substances that eliminate hydroxyl radicals produced in the body. Water is produced by hydrogen reacting with hydroxyl radicals, and water does not produce substances harmful to a living body. Thus, an apparatus for producing hydrogen water containing hydrogen that eliminates hydroxyl radicals in the body has been proposed (for example, Patent Document 1).

However, hydrogen in hydrogen water is easily diffused into air. Thus, for taking hydrogen in the body in an amount necessary for eliminating hydroxyl radicals, it is necessary that the concentration of dissolved hydrogen in hydrogen water is kept high. Therefore, in a method in which hydrogen water is ingested, it is not easy to take hydrogen in the body in an amount sufficient to react the hydrogen with hydroxyl radicals in the body. Thus, in order to easily take hydrogen in the body, a hydrogen-containing composition containing hydrogen and a surfactant has been proposed (Patent Document 2).

Some of the present inventors have studied water decomposition due to silicon nanoparticles, and hydrogen concentrations, and have disclosed the results (Non-Patent Document 1 and Patent Document 3). In addition, some of the present inventors have disclosed a solid preparation that generates hydrogen, a method for producing the solid preparation, and a method for generating hydrogen (Patent Document 4).
Patent Document 5 discloses a method for treating or ameliorating varicocele or male infertility using anthocyanin extracted from black soybean.
Non-Patent Document 2 discloses the stimulation of human damaged sperm motility with hydrogen molecule.
Non-Patent Document 3 discloses positive effect of hydrogen on the production of testosterone hormone in male infertility.

### PRIOR ART DOCUMENTS

### PATENT DOCUMENT

Patent Document 1: Japanese Patent No. 5514140
Patent Document 2: Japanese Patent Laid-Open Publication No. 2015-113331
Patent Document 3: Japanese Patent Laid-Open Publication No. 2016-155118
Patent Literature 4: International Publication No. WO 2017/130709
Patent Document 5: US 8 980 847 B2

### NON-PATENT DOCUMENT

Non-Patent Document 1: Shinsuke MATSUDA et al., Water decomposition due to silicon nanoparticles and hydrogen concentrations, Extended Abstracts of the 62nd JSAP Spring Meeting, 2015, 11a-A27-6
Non-Patent Document 2: KUMIKO NAKATA ET AL, "Stimulation of human damaged sperm motility with hydrogen molecule", MEDICAL GAS RESEARCH, BIOMED CENTRAL LTD, LONDON, UK, (20150110), vol. 5, no. 1, page 2
Non-Patent Document 3: BEGUM RAHIMA ET AL, "Molecular hydrogen may enhance the production of testosterone hormone in male infertility through hormone signal modulation and redox balance", MEDICAL HYPOTHESES, EDEN PRESS, PENRITH, US, (20180906), vol. 121, pages 6-9

### SUMMARY OF THE INVENTION

### PROBLEMS TO BE SOLVED BY THE INVENTION

For example, when mention is made of hydrogen water that has been developed for the purpose of reducing or eliminating excess hydroxyl radicals in the body, hydrogen water is attempted to be employed to take in hydrogen for eliminating the hydroxyl radicals into the body. Even when high-concentration hydrogen water is ingested, the amount of hydrogen contained in 1 liter of hydrogen water is only 18 ml (milliliter) in terms of a gas, even in a saturated state at room temperature. In addition, the saturated water concentration of 1.6 ppm is rapidly decreased in the air, and much of hydrogen in hydrogen water is gasified in an upper digestive tract such as a stomach. This disadvantageously causes pneumophagia (so-called "burp") because a sufficient amount of hydrogen is not necessarily taken in the body. When a hydrogen-containing composition with hydrogen encapsulated by a surfactant is ingested, it is necessary to ingest a large amount of the hydrogen-containing composition for taking a sufficient amount of hydrogen in the body. In addition, there may arise the above-mentioned problem that hydrogen is released in the stomach. Furthermore, even if hydrogen is taken into the body and transported to each organ, the hydrogen concentration in the organ returns to the concentration before the ingestion of hydrogen water after elapse of about 1 hour. Therefore, since the hydroxyl radicals are always produced by respiration and/or metabolism, the effect of ingesting the hydrogen water is limited.

On the other hand, Patent Document 4 discloses that hydrogen water helps to easily ingest an amount of hydrogen sufficient to eliminate hydroxyl radicals in the body into the body, but Patent Document 4 does not disclose an effect on an organ related to reproductive disorder of an animal including a human or a specific disease related to reproductive disorder of an animal including a human.

Therefore, it can be said to be still halfway to attain the development of a therapeutic agent or preventive agent for radical cure of reproductive disorder which is one of specific diseases of animals including humans, and the simplification, low cost, high functionality, and/or more accurate safety of the producing step of the therapeutic agent or preventive agent for the kidney disease.

### SOLUTIONS TO THE PROBLEMS

The present invention solves the above-mentioned technical problems. The present invention utilizes a drug as defined in the appended claims, having a hydrogen-generating ability to largely contribute to the attainment of the prevention or treatment of reproductive disorder which is varicocele different from means or approaches employed so far.

The present inventors found that, even if the above-mentioned silicon fine particle or crystal grain of silicon as defined in the appended claims are brought into contact with a water-containing liquid having a very low pH value (that is, strongly acidic) such as stomach acid, the silicon fine particle or the crystal grain of silicon as defined in the appended claims hardly generate hydrogen, but the silicon fine particle, or the crystal grain of silicon can significantly generate hydrogen when being brought into contact with a portion or a water-containing liquid that has a pH value in a neutral numerical range (including a pH value of 6 to 7 in the present application) to an alkaline numerical range. Based on these facts, the present inventors repeatedly conducted experiments and analyses in order to investigate the possibility that the above-mentioned silicon fine particle, or crystal grain of silicon contribute to the prevention or treatment of the reproductive disorder. As a result, significant effects on reproductive disorder which is varicocele could be confirmed. The "water-containing liquid" in the present application includes water itself and human body fluid.

A hydrogen generation mechanism by the reaction of the silicon fine particle or the crystal grain of silicon with water molecules is represented by the following formula (Chemical Formula 1). However, as described above, the present inventors found that the reaction represented by the formula (chemical formula 1) is a limited reaction when the silicon fine particle or the crystal grain of silicon are brought into contact with a water-containing liquid having a low pH value (typically a pH value of less than 5), but the reaction proceeds when the silicon fine particle or the crystal grain of silicon are brought into contact with a water-containing liquid having a pH value of 6 or more (particularly, more than 6). Therefore, it was very interestingly clarified that even a water-containing liquid that is weakly acidic and has a pH value of 6 allows effective generation of hydrogen. The present inventors found by further examination that, in order to promote the generation of hydrogen, it is effective to bring the silicon fine particle or the crystal grain of silicon into contact with more suitably a water-containing liquid having a pH value of 7 or more (or more than 7), still more suitably a water-containing liquid having a pH value of more than 7.4, and very suitably a water-containing liquid that is basic (hereinafter referred to as "alkaline") and has a pH value of more than 8. In the present application, a basic water-containing liquid in a basic or biocompatible range of an intestinal fluid determines the upper limit of the pH value.

(Chemical Formula 1) Si + 2H₂O → SiO₂+ 2H₂

Studies conducted by the inventors of the present application have revealed depending on conditions, contact of silicon fine particles or crystal grains of silicon (hereinafter, also referred to as "silicon fine particles or the like") with a water-containing liquid corresponding to intestinal fluid in an environment similar to that of the intestine can cause generation hydrogen in an extremely large amount of about 400 mL from 1 g of the silicon fine particles or the like. The inventors of the present application have considered that, for example, when a large amount of hydrogen is generated in the intestine, the hydrogen is efficiently absorbed into one or more tissues related to reproductive disorder by blood flow and/or direct permeation, and thus the inventors have made an attempt to utilize the above-described silicon fine particles or crystal grains of silicon for reproductive disorder which is varicocele.

When attention is paid to varicocele which is a reproductive disorder and one of the causative diseases of male infertility, varicocele can form a hypoxic condition in the testis due to an increase in temperature in the scrotum and/or stagnation of blood flow due to varicose veins. When the hypoxic condition continues, a phenomenon of deterioration in spinning function (reduced sperm concentration, reduced sperm motility and/or increased sperm malformation rate) can occur in the testis, and testicular atrophy can occur.

Here, the inventors of the present application have intensively conducted studies and analyses based on the idea that, if an increase in hydroxyl radicals in one or more tissues in which a hypoxic condition in the testis is related to reproductive disorder which is varicocele can cause damage to the tissues or damage to cells in the tissues, prevention or suppression of generation of hydroxyl radicals in the tissues leads to prevention or treatment of the reproductive disorder which is varicocele.

As a result, the present inventors have found that by ingesting or administering a preparation containing silicon fine particles having a hydrogen-generating ability, or crystal grains of silicon as defined in the claims, a decrease in the spermatogenic function due to varicocele which is a reproductive disorder can be significantly suppressed, the spermatogenic function can be markedly improved or high sperm motility can be acquired on the basis of observation of semen without undergoing surgical therapy. This result is a finding that can lead to surpassing the improving effect on the spinning function and/or the sperm motility, which are obtained by the conventional surgical therapy. The present invention has been made based on the above-mentioned viewpoint.

A drug of the present invention is a drug for a reproductive disorder which is varicocele, containing a silicon fine particle, or a crystal grain of silicon having a hydrogen-generating ability as defined in the appended claims.

In the drug, hydrogen generated from the above-mentioned physiologically acceptable silicon fine particle, crystal grain of silicon (particle size of 1 µm to 2 µm), or a silicon particle of 1 µm or more having a surface area equivalent to that of the silicon fine particle is considered to contribute to reduction in hydroxyl radicals in one or more tissues related to reproductive disorder which is varicocele. At present, the detailed mechanism is not clear, but such reduction in hydroxyl radicals is considered to be able to contribute to the prevention and/or treatment of reproductive disorder which is varicocele. A specific effect of the prevention, treatment, or improvement of reproductive disorder which is varicocele can be exhibited by the drug.

Even when the above-mentioned drug is brought into contact with a water-containing liquid having a very low pH value such as stomach acid, the drug hardly generates hydrogen. However, when the drug is brought into contact with a portion or a water-containing liquid that has a pH value in neutral to alkaline numerical ranges, the ability to significantly generate hydrogen can significantly contribute to the prevention, treatment, or improvement of reproductive disorder which is varicocele.

In the actual use of the above-mentioned drug, the use of at least one kind selected from the group of fine particles of 100 nm² or more (or more than 100 nm²) that have a surface area equivalent to that of the silicon fine particle, and are porous, polycrystalline, or unfree, hardly crushed or granulated with an undecomposed binding agent, and porous crystal grains can also be employed as an aspect.

Varicocele is a reproductive disorder of male infertility. Examples of male infertility not part of the present invention are spermatogenic dysfunction, seminal tract obstruction and/or sexual dysfunction. Typical examples of the "spermatogenic dysfunction" include oligospermia, asthenospermia, aspermia and teratozoospermia. Typical examples of the female factor (or "female infertility") which are not part of the present invention include a variety of disorders such as polycystic ovary syndrome, implantation disorder, infertility and conception disorder. There are also fertilization disorder that can be caused by both male and female factors, which are not part of the present invention.

In addition, in the present application, the expression "crystallite" is employed rather than the expression "crystal grain (or crystal particle)" when the diameter of a crystal is in the "nm order." Meanwhile, the expression "crystal grain (or crystal particle)" is employed when the diameter of a crystal is in the "µm order." In the present application, the expression "grain" is employed as the expression regardless of the presence or absence of crystallinity.

Here, the "silicon fine particle" in the present application includes a "silicon nanoparticle" having an average crystallite diameter in the nm order, specifically a crystallite diameter of substantially 1 nm or more and 100 nm or less. In a narrower sense, the "silicon fine particle" in the present application includes, as main particles, a silicon nanoparticle having an average crystallite diameter at a nano level, specifically a crystallite diameter of 1 nm or more and 50 nm or less. Here, according to the present inventors, a silicon nanoparticle having a main crystallite diameter of 1 nm or more and less than 30 nm is the "silicon fine particle" that attains the finest division as one employable aspect. In the present application, the silicon fine particle includes not only individually dispersed silicon nanoparticles, but also silicon nanoparticles in a state of an aggregate that is formed by natural gathering of a plurality of the silicon nanoparticles and has a size close to a µm size (generally 0.1 µm or more), fused silicon nanoparticles, and granulated silicon nanoparticles that cause no redispersion. The "silicon fine particle" in the present application may include porous silicon.

In the present application, a lump solid preparation that is obtained by artificially putting the silicon fine particles together through addition of a binding agent, compression, or the like and has such a size to be picked up by human fingers is sometimes referred to as "solid preparation" for discriminating the solid preparation from the "aggregate" and the "crystal grain." The "drug" in the present application includes a "solid preparation". Typical examples of the "solid preparation" include tablets, and granules or a powdered drug that assume a powdery form rather than a lump form.

In the present application, the expression "disorder" includes "malady", "disease", and "disorder" meanings. The "physiologically acceptable base material (substance or material)" in the present application is a base material (substance or material) that is substantially innocuous and causes substantially no side effect or harmful reaction even when brought into contact with the skin or the mucous membrane. The term "physiologically" includes a "medical" meaning.

### EFFECTS OF THE INVENTION

The drug of the present invention, hydrogen generated from the above-mentioned physiologically acceptable silicon fine particle as defined in the appended claims can contribute to the prevention and/or treatment of reproductive disorder which is varicocele.

### BRIEF DESCRIPTION OF THE DRAWINGS

Fig. 1 shows photographs of a normal feed (a) and a preparation (feed) (b) of a first embodiment.
Fig. 2 is a graph showing amounts of hydrogen generated in Examples 1 and 2.
Fig. 3(a) is a graph showing the time change of an amount of dissolved hydrogen generated by bringing each type of silicon fine particles of the first embodiment subjected into contact with an aqueous solution obtained by dissolving sodium hydrogen carbonate in pure water, and Fig. 3(b) is a graph showing the time change of an amount of dissolved hydrogen per gram of the silicon fine particles of the first embodiment.
Fig. 4 is a graph showing the relationship between a hydrogen generation amount (ppm) and a reaction time (minute) of each of samples A to D of the first embodiment.
Fig. 5 is a graph showing a hydrogen concentration (ppb) in 200 µl (microliter) of blood when an SD rat aged 6 weeks is caused to ingest a normal feed or a preparation (feed) of the first embodiment for 8 weeks.
Fig. 6 is a graph showing a hydrogen concentration (ppb) in exhaled air when an SD rat aged 6 weeks is caused to ingest a normal feed or a preparation (feed) of the first embodiment for 8 weeks.
Fig. 7 is a graph showing the results of a BAP test (evaluation test of antioxidative potency of blood plasma) for measuring an antioxidation degree when an SD rat aged 6 weeks is caused to ingest a normal feed or a preparation (feed) of the first embodiment for 8 weeks.
Fig. 8 shows a flow of preparation of a varicocele rat model for examining the effect of the preparation of the first embodiment.
Fig. 9 is a schematic diagram for illustrating a procedure for preparation of a varicocele rat model.
Fig. 10 shows graphs in which a varicocele rat model group is compared with a sham surgery group for the sperm concentration in Fig. 10(a) and the sperm motility in Fig. 10(b).
Fig. 11 is a schematic diagram for illustrating a site for measurement of an internal spermatic vein diameter in an iliopsoas vein intersection.
Fig. 12 shows implementation plans (protocols) for examining preventive and therapeutic effects of the preparation of the first embodiment in (1) Comparative Example and (2) the present embodiment.
Fig. 13 shows graphs in which Comparative Example is compared with Example for the sperm concentration in Fig. 13(a) and the sperm motility in Fig. 13(b).
Fig. 14 is a graph in which Comparative Example is compared with Example using the positive rate (%) of oxidative stress marker "8-OHdG" of one DNA as an index.

### DESCRIPTION OF REFERENCE SIGNS

100: Preparation

### EMBODIMENTS OF THE INVENTION

Embodiments of the present invention will be described in detail with reference to the accompanying drawings.

### <First Embodiment>

A preparation of the present embodiment contains a silicon fine particle (hereinafter, also collectively referred to as "silicon fine particle") or a crystal grain of silicon having a hydrogen-generating ability as defined in the appended claims. Hereinafter, the silicon fine particle, and the solid preparation containing the silicon fine particle or the crystal grain as an example of a "drug" of the present embodiment will be described in detail. In addition, a production method for producing a drug of the present embodiment, or a production method for producing a substance of the drug which both do not form part of the present invention will also be described in detail.

### [1] Silicon Fine Particle, Crystal Grain of Silicon, Preparation, and Production Method Therefor

The preparation (solid preparation as a typical example) according to the present embodiment is produced using a silicon fine particle that may contain a silicon nanoparticle (hereinafter, also typically referred to as "silicon fine particle") obtained by finely dividing, according to a bead mill method, a commercially available high-purity silicon particle powder (typically, manufactured by Kojundo Chemical Laboratory Co., Ltd., particle diameter distribution: < ϕ 5 µm (but silicon particles having a crystal grain diameter of more than 1 µm, purity: 99.9% i-type silicon) as a silicon particle. The present embodiment employs a step of forming the silicon fine particle by grinding a silicon particle in an ethanol solution. The method disclosed in the present disclosure is not limited to the above-described method.

Specifically, 200 g of the high-purity silicon powder is dispersed in 4 L (litters) of a 99.5 wt% ethanol solution using a bead mill apparatus (manufactured by AIMEX CO., Ltd. horizontal continuous ready mill, (model: RHM-08), and ϕ 0.5 µm zirconia beads (volume: 750 ml) are added thereto. The mixture is finely divided by performing grinding (one-step grinding) at a rotation speed of 2500 rpm for 4 hours. In the present disclosure, grinding in which the size and/or type of the beads are/is appropriately changed may be employed for the purpose of adjusting a required particle size or particle size distribution or the like. Therefore, the apparatus and method disclosed in the present disclosure are not limited.

In the present disclosure, a separation slit provided in a grinding chamber of the bead mill apparatus separates the mixture into the beads and an ethanol solution containing silicon fine particles. The ethanol solution containing silicon fine particles that has been separated from the beads is heated to 30°C to 35°C with a vacuum evaporator. As a result, the ethanol solution is evaporated to provide the silicon fine particle.

The silicon fine particle obtained by the method and capable of serving as a substance of a drug of the present embodiment mainly contain a silicon fine particle having a crystallite diameter of 1 nm or more and 100 nm or less. More specifically, as a result of measuring the silicon fine particle by an X-ray diffractometer (SmartLab manufactured by Rigaku Corporation), the following values were obtained as one example. In a volume distribution, the mode diameter was 6.6 nm; the median diameter was 14.0 nm; and the average crystallite diameter was 20.3 nm.

The silicon fine particle was observed using a scanning electron microscope (SEM), and the result showed that the silicon fine particles were partially aggregated to form a slightly large formless aggregate of about 0.5 µm or less. Individual silicon fine particles were observed using a transmission electrode microscope (TEM), and the result showed that main silicon fine particles had a crystallite diameter of about 2 nm or more and about 30 nm or less.

Thereafter, a first mixing step of mixing hydrogen peroxide water with the silicon fine particles in a glass container (hereinafter, also referred to as a "H₂O₂ treatment" or a "hydrogen peroxide water treatment step") is performed in the present disclosure. In the present disclosure, the temperature of the hydrogen peroxide water (3.5 wt% in the present embodiment) in the mixing step is 75°C. The mixing time is 30 minutes. A sufficient stirring treatment in the first mixing step (hydrogen peroxide water treatment step) is preferred to increase the opportunity of the silicon fine particles being brought in contact with the hydrogen peroxide water. Even when the temperature of the hydrogen peroxide water in the first mixing step (hydrogen peroxide water treatment step) is, for example, about room temperature, at least a part of the effects of the present disclosure can be exhibited. The silicon fine particles that have been subjected to the first mixing step can also serve as the substance of the drug of the present invention as defined in the claims.

The silicon fine particles mixed with the hydrogen peroxide water are subjected to a solid-liquid separation treatment using a known centrifugal separator to remove the hydrogen peroxide water and thus provide silicon fine particles. As a result, it is possible to obtain silicon fine particles having surfaces treated with hydrogen peroxide water. Here, the treatment of the surfaces of the silicon fine particles with hydrogen peroxide water can remove an alkyl group (for example, a methyl group) present on the surfaces of the silicon fine particles. As a result, the silicon fine particles can form a state where they have surfaces capable of being brought into direct contact with a medium capable of containing a water-containing liquid, while as a whole retaining hydrophilicity on their surfaces. Such a special surface treatment can promote the generation of hydrogen with higher accuracy. The hydrogen can contribute to prevention and/or treatment of reproductive disorder which is varicocele because the hydrogen can contribute to reduction in hydroxyl radicals in one or more tissues related to reproductive disorder which is varicocele.

Thereafter, a second mixing step of mixing the silicon fine particles with an ethanol solution is further performed in the present disclosure. A sufficient stirring treatment in the mixing step is preferred to increase the opportunity of the silicon fine particles being brought into contact with the ethanol solution (99.5 wt% in the present embodiment). The silicon fine particles mixed with the ethanol solution are subjected to a solid-liquid separation treatment using a known centrifugal separator for removing the ethanol solution that is highly volatile, followed by sufficiently drying to produce silicon fine particles. The silicon fine particles that has been subjected to the second mixing step can also serve as the substance of the drug of the present invention as defined in the claims.

In the present invention, as another type of final silicon fine particles as defined in the appended claims, silicon fine particles were also produced, with the mixing time of the hydrogen peroxide water with the silicon fine particles set to 60 minutes in the first mixing step of the above-mentioned steps. By adjusting the treatment time of the bead mill method, and the like, in place of the silicon fine particle, a crystal grain of silicon (for example, a crystal grain of silicon that substantially contains no "silicon fine particles"' of 1 nm or more and 100 nm or less) can also be produced through at least the above-mentioned first mixing step. The above-mentioned crystal grains of silicon can also serve as the substance of the drug of the present invention as defined in the appended claims.

In the present disclosure, isopropyl alcohol or hydrofluoric acid (aqueous solution) is not used. The present disclosure uses the ethanol solution and the hydrogen peroxide water in order to obtain the silicon fine particles (or the aggregates thereof) or the crystal grains of silicon, and thus, it is worth noting that it is possible to provide a drug (or a preparation) that is safer and more secure for a living body, a production method for producing the drug (or the preparation), or a production method for producing a substance of the drug (or the preparation).

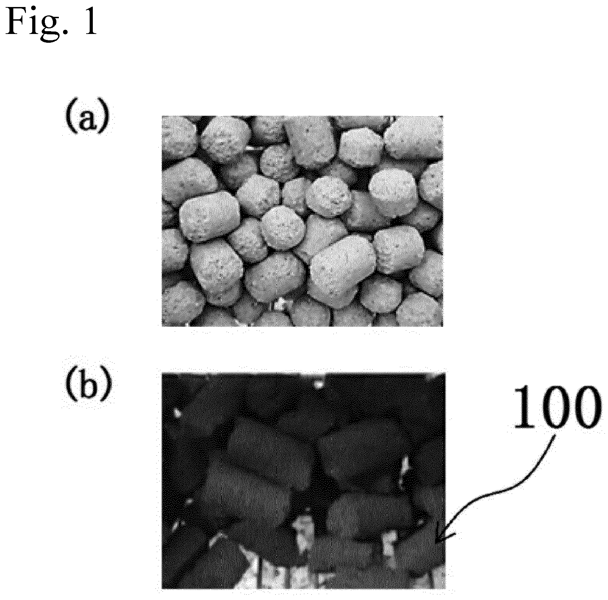

The present disclosure further produce a preparation (typically a solid preparation) containing the silicon fine particles (including the aggregates thereof) and/or the crystal grains of silicon that can serve as the substance of the drug of the present invention. The preparation of the present disclosure is an experimental sample (or feed) described later. Fig. 1(a) is an overview photograph of a normal feed as Comparative Example, and Fig. 1(b) is an overview photograph of a normal feed which contains fine particles (and/or silicon crystal grains) and in which silicon fine particles (and/or silicon crystal grains) are kneaded. The amount of the silicon fine particles (and/or silicon crystal grains) contained in the feed can be appropriately changed according to various conditions such as the weight or diathesis of a human or a non-human ingesting the feed, or ease of ingestion in the case of oral administration.

In the present disclosure, thereafter, a pH value adjusting agent adding step is performed, in which 97.5 wt% of a normal feed (manufactured by Oriental Yeast Co., Ltd., model number: AIN93M) as a base material, and 2.5 wt% of the above-mentioned produced silicon fine particles (and/or crystal grains of silicon) are kneaded using a known kneader together with an aqueous solution of citric acid as a pH value adjusting agent. The amount of the aqueous solution of citric acid is not particularly limited, but for example, about 0.5 wt% of the aqueous solution with respect to the total amount of the silicon fine particles (and/or the crystal grains of silicon) and the feed can be selected. The pH value of the aqueous solution of citric acid is about 4. The type of the base material is not limited as long as it is a physiologically acceptable base material (substance or material).

Thereafter, the feed obtained by kneading the silicon fine particles (and/or crystal grains of silicon) is molded using a commercially available pelleter. Thereafter, moisture is removed using a dryer heated to about 90°C, and the size of the molded product is selected using a sieve, whereby a preparation (feed) 100 shown in Fig. 1(b) can be produced. Thereafter, from the viewpoint of preventing or inhibiting the preparation (feed) 100 from being brought into contact with moisture, one suitable aspect is storing the preparation (feed) 100 in a packaged state. An aspect in which the silicon fine particles (including the aggregates thereof) that do not form the preparation 100 are contained in a known material (base material) other than the normal feed is also one employable aspect.

Here, citric acid contained in the preparation 100 of the present embodiment can serve as a pH value adjusting agent that sets the pH value of the water-containing liquid when the preparation 100 is disintegrated in pure water to 4 or more and less than 7 (more narrowly, 6 or less). As a result, the adjustment action of the pH value by citric acid that sets the water-containing liquid to an acidic state can prevent or inhibit the generation of hydrogen due to the preparation 100 being brought into contact with moisture and the like in the outside air. Therefore, as one suitable aspect of the present embodiment, the preparation 100 contains the citric acid. Even when the preparation of the present embodiment does not contain the citric acid, at least a part of the effects of the present embodiment can be exhibited.

### <Modified Example (1) of First Embodiment>

In the production method for producing the drug (or preparation 100) of the first embodiment, or the production method for producing the substance of the drug (or preparation 100), one suitable aspect is further including an introduction step of introducing a "pH adjusting agent" into the drug (or preparation). The pH adjusting agent makes adjustment so as to satisfy a condition where hydrogen is more likely to be generated in an appropriate environment in the body, in other words, so as to set a pH value in a numerical value in which hydrogen is more likely to be generated.

The citric acid in the first embodiment is one example of the "pH adjusting agent," but the "pH adjusting agent" is not limited to the citric acid. The material for the "pH adjusting agent" is not limited as long as it is a material (hereinafter, also referred to as an acid agent) allowing adjustment to acidity having a pH value of 2 or more (more preferably 3 or more) and less than 7 (more preferably 6 or less). A typical example of the acid agent is at least one selected from the group consisting of citric acid, gluconic acid, phthalic acid, fumaric acid, and lactic acid, or a salt thereof. A typical example of the alkaline agent is sodium hydrogen carbonate. One suitable aspect is employing a material widely used as a food additive and having advantages such as excellent safety and versatility.

### <Modified Example (2) of First Embodiment>

One suitable aspect is preparing the drug (or preparation 100) of the first embodiment so as to be suitable for oral ingestion in order to improve the taking property of the silicon fine particles or crystal grains of silicon of the first embodiment. For example, another suitable aspect is employing a known jelly preparation of the preparation 100, or employing known preparations such as a microgranule agent, a liquid agent, a dry syrup agent, a chewable agent, a troche agent, and a fine granule agent and the like.

It is preferable to prepare each of the above-mentioned preparations so as to have a sustained release property because hydrogen is more likely to be generated in an appropriate environment in the body (for example, in an intestinal tract that is downstream from a stomach). Specifically, as one suitable aspect, the preparation 100 has a sustained release property for generating hydrogen in the intestinal tract (for example, in the whole intestinal tract) to exhibit a pharmacological function. Examples of other means for exhibiting a sustained release property include adjustment of the particle size distribution of silicon fine particles, adjustment of a coating material, and/or storage of the solid preparation in a capsule (including a microcapsule) that can function as a sustained-release agent described later. The form of the preparation is not also particularly limited, and the preparation can be employed in a wide form. Therefore, the dosage form and form of the drug (or preparation 100) for reproductive disorder of the present embodiment are not limited to the above-mentioned dosage forms and forms.

### <Modified Example (3) of First Embodiment>

In the present embodiment, the same high-purity silicon particle powder as that used in the first embodiment (typically, silicon particles having a crystal grain diameter of more than 1 µm) is ground in one step by the procedures described in the first embodiment. In the present embodiment, the ϕ 0.5 µm zirconia beads (volume: 750 ml) used in the one-step grinding are automatically separated from a solution containing silicon fine particles in a bead mill grinding chamber. Furthermore, ϕ 0.3 µm zirconia beads (volume: 300 ml) are added to the solution containing silicon fine particles from which the beads have been separated, and the mixture is finely divided by performing grinding (two-step grinding) at a rotation speed of 2500 rpm for 4 hours.

The silicon fine particles and/or silicon crystal grains containing the beads are separated from the solution containing silicon fine particles and/or silicon crystal grains as described above. The ethanol solution containing silicon fine particles and silicon crystal grains, which has been separated from the beads, is heated to 40°C using a vacuum evaporator in the same manner as in the first embodiment, whereby the ethanol solution is evaporated to obtain the silicon fine particles and/or silicon crystal grains.

### <Modified Example (4) of First Embodiment>

Another employable aspect is also further providing a physiologically acceptable covering layer that covers the preparation 100 according to the first embodiment or the preparations described in the Modified Examples (1) and (2) of the first embodiment. For example, it is possible to employ a known enteric material hardly soluble in the stomach as a coating agent that covers the outermost layer of the preparation 100. An example of a physiologically acceptable covering layer applicable as a capsule preparation is a capsule that encapsulates the silicon fine particle (mainly the aggregate of silicon fine particles) or the crystal grain of silicon and is produced from a known enteric material hardly soluble in the stomach. When the preparation 100 is employed, a disintegrating agent may be further contained. For the disintegrating agent, a known material can be employed. In addition, an example of a more suitable disintegrating agent is an organic acid, and the most suitable example is citric acid. Here, the organic acid can also function as a binding agent that brings the silicon fine particles and/or silicon crystal grains into a lump form.

In addition, the temperature condition of the water-containing liquid for generating hydrogen in each of the above-mentioned embodiments is not limited. The water-containing liquid capable of generating hydrogen and having a temperature of suitably 30°C (more suitably 35°C) or higher and 45°C or lower promotes the reaction of generating hydrogen.

### <Examples>

Hereinafter, the above-mentioned embodiments will be described in more detail by way of Examples, but the embodiments are not limited to these examples.

### (Example 1)

The present inventors examined the state of generation of hydrogen without performing a molding step using a pelleter, to evaluate silicon fine particles themselves. Specifically, an experiment was conducted as Example 1, using silicon fine particles subjected to the one-step grinding in the first embodiment.

The silicon fine particles described in the first embodiment in an amount of 10 mg and in a form of a powdered drug (that is, citric acid was not mixed or kneaded) were poured into a glass bottle having a volume of 100 ml (borosilicate glass having a thickness of about 1 mm, LABORAN Screw Tubular Bottle manufactured by AS ONE Corporation). Tap water having a pH value of 7.1 in an amount of 30 ml was poured into the glass bottle. The glass bottle was hermetically sealed under the temperature condition of a liquid temperature of 25°C. The concentration of hydrogen in the liquid in the glass bottle was measured. The hydrogen generation amount was determined using the measured concentration of hydrogen. For measurement of the concentration of hydrogen, a portable dissolved hydrogen meter (Model: DH-35A manufactured by DKK-TOA CORPORATION) was used.

### (Example 2)

Example 2 was conducted in the same manner as Example 1 except that 30 ml of tap water was poured and the temperature condition was changed to a liquid temperature of 37°C.

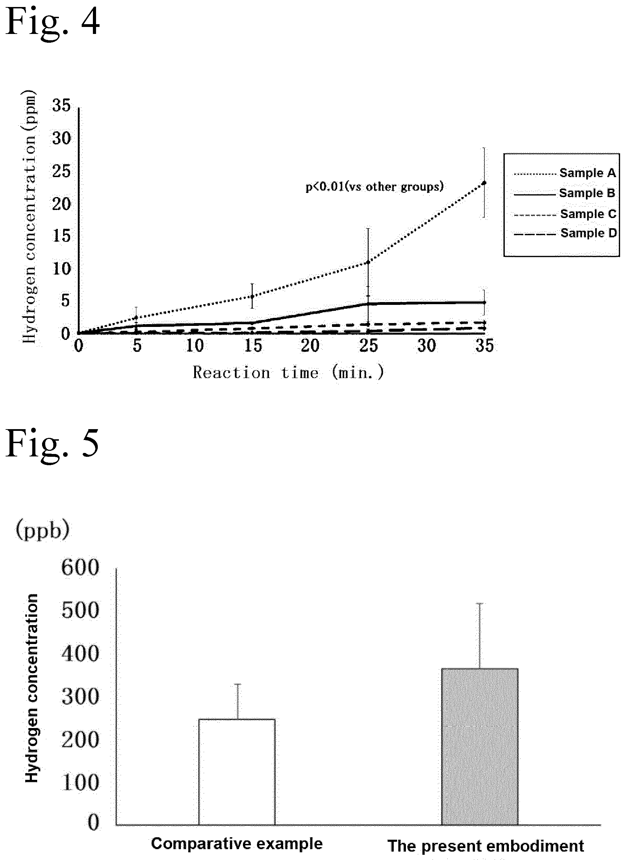

Fig. 4 shows the results of Examples 1 and 2. In Fig. 4, the abscissa axis represents the time (min) during which the preparation 100 is kept in contact with the water-containing liquid, and the ordinate axis of the graph represents the hydrogen generation amount.

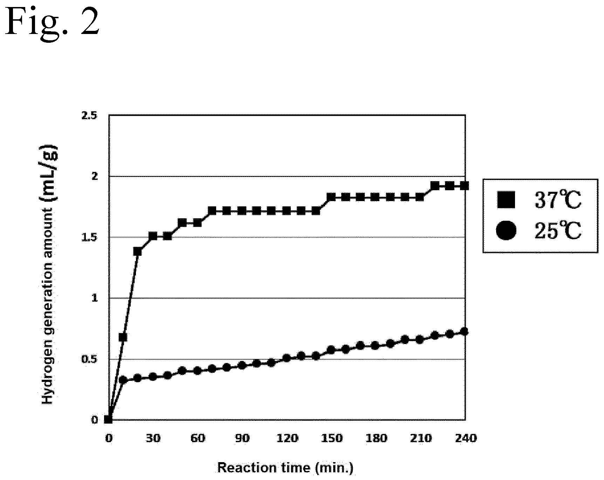

As shown in Fig. 2, the generation of hydrogen was confirmed even when nearly neutral water was brought into contact with the silicon fine particles described in the first embodiment. It was also clarified that a high liquid temperature increases the hydrogen generation amount. Particularly, it was confirmed that, when the liquid temperature is 37°C close to human body temperature, the generation of hydrogen is attained in a shorter time and a great amount (1.5 ml/g or more) of hydrogen is continuously generated thereafter.

According to further studies provided by the present inventors, it was clarified that the silicon fine particles or the crystal grains of silicon have a hydrogen-generating ability of 5 ml/g or more when they are brought into contact with a water-containing liquid having a pH value of 6 or more and less than 7, and have a hydrogen-generating ability of 10 ml/g or more when they are brought into contact with a water-containing liquid having a pH value of more than 7 and less than 10. It was also clarified that the silicon fine particles or the crystal grains of silicon have a hydrogen-generating ability of 2 ml/g or less when they are brought into contact with a water-containing liquid having a pH value of 1 or more and 3 or less. This means that the silicon fine particles or the crystal grains of silicon have almost no hydrogen-generating ability, for example, in a human stomach (stomach acid), and exhibit a hydrogen-generating ability in contact with, for example, a water-containing liquid in an intestine (typically, intestinal fluid) that is downstream from the stomach, whereby the generation of hydrogen at an appropriate place in the human body can be attained.

### <Experiment of Measuring Amount of Hydrogen Generated by Contact Between Silicon Fine Particles and Water-Containing Liquid>

The present inventors also examined the time change of an amount of hydrogen generated by bringing the same silicon fine particles as the silicon fine particles (not the solid preparation) of the first embodiment except that citric acid is not contained into contact with an aqueous solution obtained by dissolving sodium hydrogen carbonate in pure water.

Specifically, 11 mg of the silicon fine particles (first mixing step: 30 minutes) or 5 mg of the silicon fine particles (first mixing step: 60 minutes) are mixed in a glass container with an aqueous solution having sodium hydrogen carbonate (1.88 wt%) dissolved therein. The aqueous solution has a pH of about 8.3. Thereafter, the glass container was filled to its opening with the aqueous solution and covered with a lid so as not to allow entry of air for complete hermetic sealing.

The lid is made of polypropylene, but a multilayer filter of polyethylene and polypropylene was used as an inner lid, whereby transmission and leakage of generated hydrogen can be sufficiently inhibited. Some time later after the hermetic sealing, the silicon fine particles prepared of the present embodiment are visually confirmed to be evenly mixed in the whole aqueous solution from their appearance.

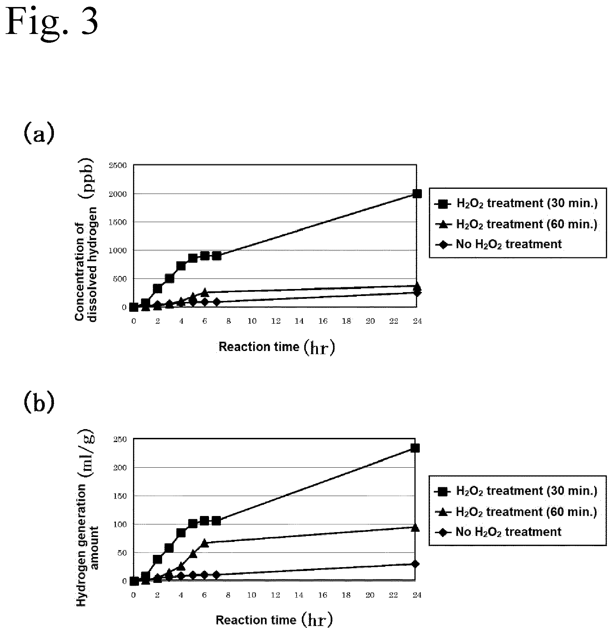

Fig. 3(a) is a graph showing the time change of a concentration of dissolved hydrogen generated by bringing each type of the silicon fine particles (not the solid preparation) of the first embodiment into contact with an aqueous solution (pH value = 8.3) obtained by dissolving sodium hydrogen carbonate in pure water. Fig. 3(b) is a graph showing the time change of a hydrogen generation amount per gram of each type of the silicon fine particles. For reference, the graphs show the results of using the silicon fine particles not subjected to the first mixing step. The amounts of dissolved hydrogen were measured using a portable dissolved hydrogen meter (manufactured by DKK-TOA CORPORATION, model: DH-35A).

As shown in Figs. 3(a) and 3(b), it was clarified that the first mixing step promotes the generation of hydrogen. Particularly, as shown in Fig. 3(b), it is worth noting that the first mixing step is performed to continuously provide a hydrogen generation amount of 40 ml or more in 2 hours after elapse of 2 hours from the start of generation of hydrogen.

The hydrogen generation amount of the silicon fine particles subjected to the first mixing step with a mixing time of 60 minutes is considered to be smaller than the hydrogen generation amount of the silicon fine particles with a mixing time of 30 minutes due to the difference in thickness of an oxide film on the surfaces of the silicon fine particles. That is, it is considered that the silicon fine particles subjected to the first mixing step with a mixing time of 60 minutes has a thicker oxide film, which makes it difficult to bring the silicon fine particles into direct contact with the medium (aqueous solution) to inhibit the generation of hydrogen.

According to further research and analyses by the present inventors, the silicon fine particles can attain sufficient surface areas capable of being brought into direct contact with the medium, while appropriately retaining hydrophilicity of the surfaces thereof, when subjected to the first mixing step with a mixing time of more than 2 minutes and 50 minutes or less (more suitably 3 minutes or more and 40 minutes or less, more suitably 4 minutes or more and 30 minutes or less, most suitably 5 minutes or more and 20 minutes or less). As a result, the generation of hydrogen can be more accurately promoted with the mixing time fallen within the above-mentioned range.

### <Experiment of Measuring Amount of Hydrogen Generated by Contact Between Preparation and Water-Containing Liquid>

In addition to the above-mentioned results of Examples 1 and 2, the present inventors evaluated the hydrogen generation amounts (ppm) related to the following four samples A to D under different conditions for the preparation 100 of the first embodiment subjected to molding by a pelleter.

### (Example 3)

A sample A is obtained by charging 200 mg of a ground product obtained by grinding the preparation once molded by a pelleter into 2 ml of a water-containing liquid having a pH value of 8.2. (water-containing liquid: pure water)
A sample B is obtained by charging 200 mg of the preparation into 2 ml of a water-containing solution having a pH value of 8.2. (water-containing liquid: pure water)
A sample C is obtained by charging 200 mg of a ground product obtained by grinding the preparation once molded by a pelleter into 2 ml of pure water.
A sample D is obtained by charging 200 mg of the preparation into 2 ml of pure water.

Fig. 4 is a graph showing the relationship between a hydrogen generation amount (ppm) and a reaction time (minutes) of each of the above-mentioned samples A to D. As shown in Fig. 4, it was confirmed that the ground product of the preparation tends to have a significantly more hydrogen generation amount with the passage of time than that of the unground preparation. This suggests that, for example, the hydrogen generation amount when the preparation bitten by the human enters the body is greater than that when the human swallows the preparation as it is. The water-containing liquid having a pH value of 8.2 tended to generate more hydrogen than that of pure water, which suggested that the reaction with the intestinal fluid provides an increase in the hydrogen generation amount.

### <Effect Confirmation Experiment When Causing SD Rat Aged 6 Weeks to Ingest Normal Feed or Preparation (Feed) for 8 Weeks>

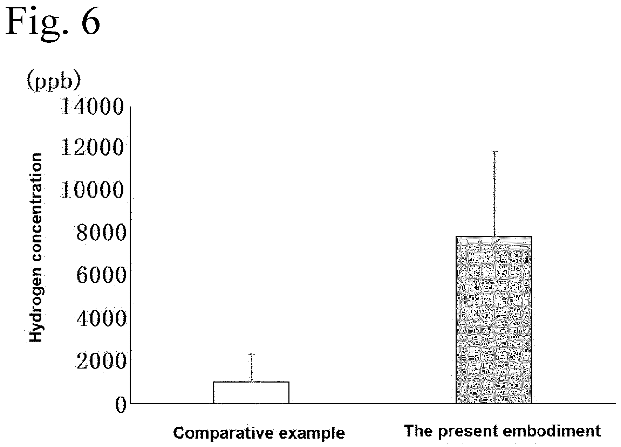

Fig. 5 is a graph showing a hydrogen concentration (ppb) in 200 µl (microliter) of blood when an SD rat aged 6 weeks (Sprague-Dawley rat, referred to as an "SD rat" in the present application) is caused to ingest a normal feed (Comparative Example in Fig. 5) or the preparation (feed) (the present embodiment in Fig. 5) for 8 weeks. In addition, Fig. 6 is a graph showing a hydrogen concentration (ppb) in exhaled air when an SD rat aged 6 weeks is caused to ingest a normal feed (Comparative Example in Fig. 6) or the preparation (feed) (the present embodiment in Fig. 6) for 8 weeks. The hydrogen concentration in the blood was measured with a sensor gas chromatograph apparatus (model: SGHA-P2 manufactured by FIS, Inc.). The hydrogen concentration in the exhaled air was similarly measured with a sensor gas chromatograph apparatus (model: SGHA-P2 manufactured by FIS, Inc.) after leaving the rat in a completely sealed container for 8 minutes.

As shown in Figs. 5 and 6, the rat (the present embodiment) ingesting the preparation (feed) was confirmed to have a higher hydrogen concentration in the blood and the exhaled air. The difference in the hydrogen concentration in the blood is considered to be relatively small because the hydrogen generated from the preparation is rapidly diffused outside the body. Hydroxyl radicals may be involved in reproductive disorder. Therefore, from the results of Figs. 5 and 6, the increase in the hydrogen concentration in the blood and/or the exhaled air suggests that the hydrogen generated from the solid preparation of the present embodiment may contribute to the effect of preventing, treating or improving reproductive disorder.

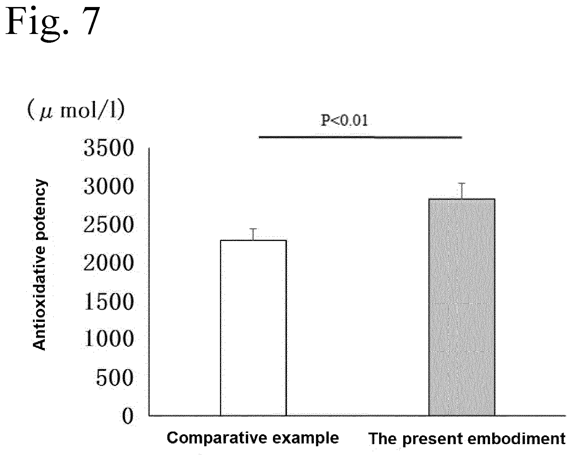

Fig. 7 is a graph showing the result of a BAP test (evaluation test of antioxidative potency of blood plasma) for measuring an antioxidation degree when an SD rat aged 6 weeks is caused to ingest a normal feed (Comparative Example in Fig. 7) or a preparation (feed) (the present embodiment in Fig. 7) for 8 weeks. The BAP test was measured with a FREE Carrio Duo apparatus (manufactured by Diacron International, model: DI-601M).

As shown in Fig. 7, the rat (the present embodiment) ingesting the preparation (feed) was confirmed to have significantly higher antioxidative potency. Therefore, it has been revealed that by administering the preparation according to the present embodiment, an effect of preventing, treating or improving reproductive disorder is exhibited.

### <Confirmation Experiment of Preventive Effect Due to Preparation of First Embodiment Using Varicocele Rat Model>

Based on each of the above-mentioned basic experiments, the present inventors conducted a confirmation experiment for a preventive and therapeutic effect due to the preparation 100 of the first embodiment using a varicocele rat model.

### (Preparation of varicocele rat model)

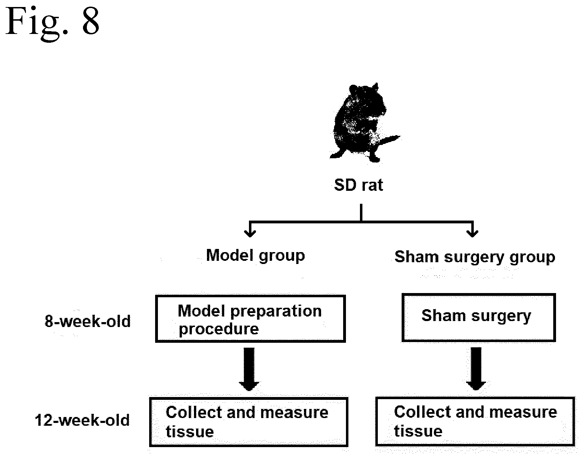

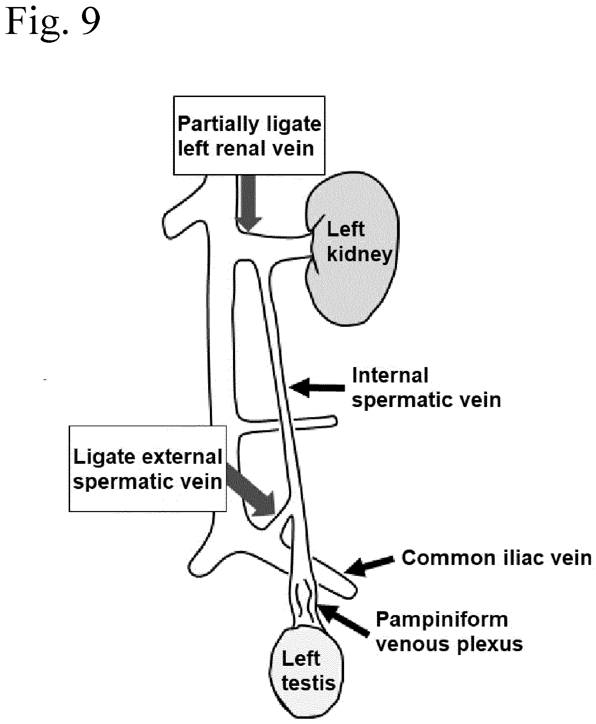

Fig. 8 shows a flow of preparation of a varicocele rat model for examining the effect of the preparation 100 of the first embodiment. Fig. 9 shows a procedure for preparation of a varicocele rat model. As shown in Fig. 9, the varicocele rat model was prepared by first partially ligating the left renal vein and further ligating the left external spermatic vein.

As shown in Fig. 8, five rats subjected to sham surgery (sham surgery group) were also prepared for confirming that the five rats of the model (model group) were properly prepared. The rats were fed with normal feed by free intake before and after the time point of 8 weeks of age at which each surgery was performed (i.e., until 12 weeks of age after birth).

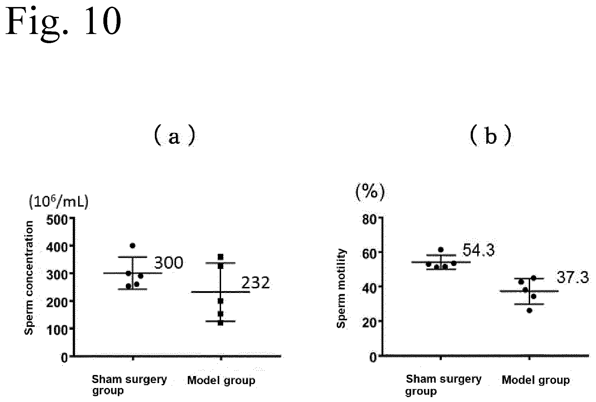

When the varicocele rat model reaches the age of 12 weeks, the present inventors evaluated the varicocele formation state by comparing the varicocele rat model with the rats subjected to sham surgery. Specifically, as shown in Fig. 10, tissues of the rats were collected and measured when the varicocele rat model reached the age of 12 weeks.

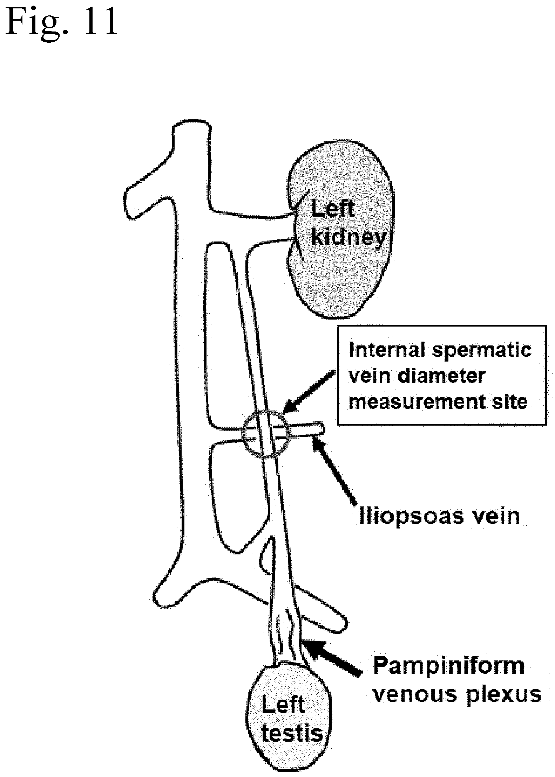

Fig. 10 shows graphs in which the above-described rat model is compared with the sham surgery group for the sperm concentration in Fig. 10(a) and the sperm motility in Fig. 10(b). In addition, Fig. 11 is a schematic diagram for illustrating a site for measurement of an internal spermatic vein diameter in an iliopsoas vein intersection.

As shown in Fig. 10, it was confirmed that the values of the sperm concentration and the sperm motility rate in the model group were significantly lower than those in the sham surgery group. In addition, it was confirmed that as shown in Fig. 11, the internal spermatic vein diameter of the varicocele rat model expanded to 0.5 mm or more when the internal spermatic vein diameter at the iliopsoas vein intersection was measured. On the other hand, the above-described expansion did not occur in the sham surgery rats. Therefore, it was confirmed that the varicocele rat model was properly prepared.

### (Example 4)

In the present experiment, the following two types of varicocele rat models ((1) Comparative Example and (2) first embodiment) were used for comparison.

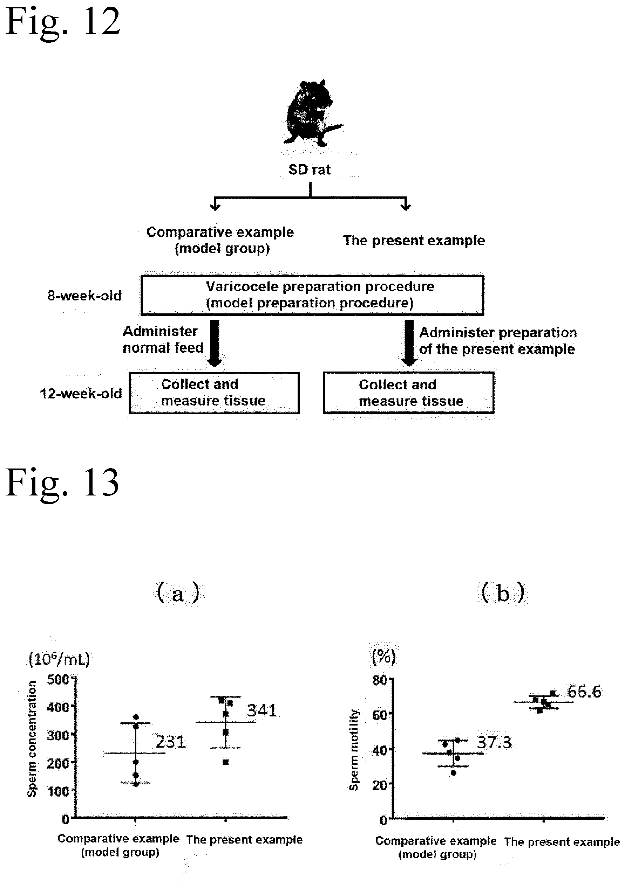

Fig. 12 shows implementation plans (protocols) for examining preventive and therapeutic effects of the preparation 100 of the first embodiment in (1) Comparative Example described below and (2) the present example. In the present example, administration was performed by free intake by SD rats.
(1) Comparative Example (control group): only a normal feed shown in Fig. 1(a) is administered after birth. An 8-week-old rat is subjected to the procedure for producing the varicocele rat model. Four weeks thereafter (i.e., from 12-week-old rats), tissues are collected and measured to acquire various data described later.
(2) The present example: normal feed is given for 8 weeks after birth. As in Comparative Example, an 8-week-old rat is subjected to the procedure for producing the varicocele rat model. After aged 8 weeks, only normal feed (hereinafter, also referred to as "feed of the first embodiment" or a "preparation") kneaded with silicon fine particles and silicon crystal grains (1 wt% in this example) shown in Fig. 1(b) is administered. As in Comparative Example, four weeks thereafter (i.e., from 12-week-old rats), tissues are collected and measured to acquire various data described later, where 8-week-old rats are used as a reference.

Fig. 13 shows graphs in which Comparative Example is compared with the present Example for the sperm concentration in Fig. 13(a) and the sperm motility in Fig. 13(b). As shown in Fig. 13, it was confirmed that the values of the sperm concentration and the sperm motility rate in the present Example were much higher than those in Comparative Example. In addition, the fact that the measured values in the present Example are higher than the measured values in the sham surgery group which do not have varicocele as shown in Fig. 10 is worth noting as an effect of the feed (preparation) of the first embodiment. Therefore, it can be said that the improving effect shown in Fig. 13 is equivalent to or higher than the improving effect obtained by surgical therapy adopted heretofore.

As described above, a significant improving effect on the sperm concentration and the sperm motility was observed without performing surgical therapy adopted heretofore, and thus, it was possible to confirm a high therapeutic effect on reproductive disorder. In addition, the results from Example 4 suggest that that a preventing effect on reproductive disorder was also exhibited because even if varicocele that can cause reproductive disorder occurred, the sperm concentration and the sperm motility rate did not decrease.

### (Example 5)

As described above, an effect of improving the antioxidant power and the sperm motility was confirmed in the rats ingesting the preparation 100, and an effect of improving the antioxidative potency and the sperm motility was also confirmed in another index shown below. "8-OHdG" is a by-product generated when DNA is oxidatively damaged by active oxygen species such as hydroxy radicals.

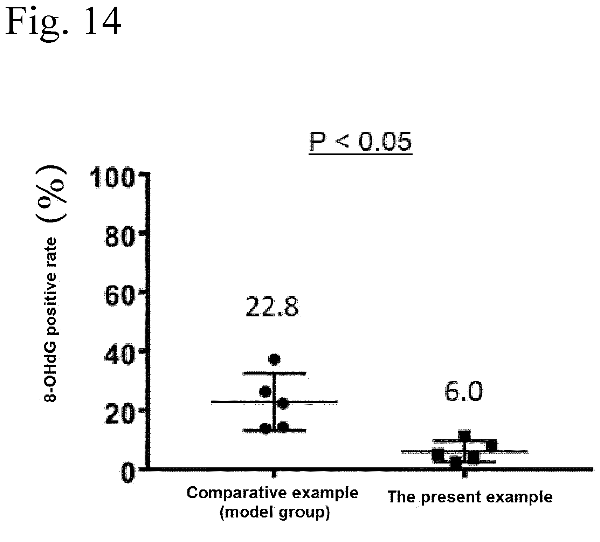

Fig. 14 is a graph in which the following two types of varicocele rat models ((1) Comparative Example and (2) first embodiment) are compared using the positive rate (%) of oxidative stress marker "8-OHdG" of one DNA in the rat as an index.
(1) Comparative Example (control group): only a normal feed shown in Fig. 1(a) is administered after birth. An 8-week-old rat is subjected to the procedure for producing the varicocele rat model. Four weeks thereafter (i.e., from 12-week-old rats), tissues are collected and measured to acquire data of the "8-OHdG" positive rate.
(2) The present example: normal feed is given for 8 weeks after birth. As in Comparative Example, an 8-week-old rat is subjected to the procedure for producing the varicocele rat model. After aged 8 weeks, only normal feed (hereinafter, also referred to as "feed of the first embodiment" or a "preparation") kneaded with silicon fine particles and silicon crystal grains (1 wt% in this example) shown in Fig. 1(b) is administered. As in Comparative Example, four weeks thereafter (i.e., from 12-week-old rats), tissues are collected and measured to acquire data of the "8-OHdG" positive rate, where 8-week-old rats are used as a reference.

As shown in Fig. 14, the already confirmed effect of improving the antioxidant potency and the sperm motility in the rat was reconfirmed in the index of "8-OHdG" positive rate.

### <Other Embodiments>

One aspect of the production method for producing silicon fine particles in the above-mentioned drug (preparation) includes a step of finely dividing silicon particle each having a crystal grain diameter of more than 1 µm by a physical grinding method to form silicon fine particles including "silicon nanoparticles" each having a crystallite diameter of 1 nm or more and 100 nm or less. A suitable example of the physical grinding method is a method for grinding a silicon particle by a bead mill grinding method, a planetary ball mill grinding method, a shock wave grinding method, a highpressure collision method, a jet mill grinding method, or a combination of two or more thereof. It is also possible to employ known chemical methods. From the viewpoint of production costs or ease of production control, a particularly suitable example is only a bead mill grinding method or a grinding method including at least a bead mill grinding method.

The above-mentioned embodiments employ, as a starting material, silicon particles, i.e., a commercially available high-purity silicon panicle powder. The starting material, however, is not limited to such silicon particles.

As one suitable aspect, the employment of porous crystal grains having nano-order voids together with or in place of the "aggregates" in each of the above-described embodiments can attain the use of particles having a large overall diameter and/or particles having a large surface area. For example, the silicon fine particle of each of the above-mentioned embodiments does not pass through a cell membrane of the intestinal tract and between cells of the intestinal tract;; or the crystal grain of silicon in each of the above-described embodiments does not pass through the cell membrane and between the cells. This is one suitable aspect from the viewpoint of securing the safety of each of the above-mentioned embodiments with higher accuracy.

In humans and non-human animals, one of causes for efficiently generating hydrogen from the silicon fine particles of each of the above-mentioned embodiments is considered to be a mildly alkaline intestinal fluid. Therefore, in each of the above-mentioned embodiments, in order to support the attainment of the mildly alkaline, for example, a mixture obtained by previously mixing the silicon fine particles with a bicarbonate such as sodium bicarbonate (sodium hydrogen carbonate) or potassium bicarbonate is administered as one of effective methods. In this case, it is preferable that the bicarbonate is decomposed by, for example, human stomach acid, so that the mixture is protected from the stomach acid to cause the mixture to serve as an enteric coating agent for dissolution in the intestine. Therefore, as one suitable aspect, the administration method is the oral administration of the enteric coating agent containing the silicon fine particle or the crystal grain of silicon having a hydrogen-generating ability and the bicarbonate in each of the above-mentioned embodiments.

The disclosure of the above-mentioned embodiments or Examples is intended for describing the embodiments and is not intended for limiting the present invention. The scope of the present invention is defined and delimited by the appended claims.

### INDUSTRIAL APPLICABILITY

A drug of the present invention as defined in the appended claims can be widely utilized in a medical industry, a medical drug industry, and a health industry.

## Claims

1. A drug for use in preventing or treating varicocele as a reproductive disorder of male infertility comprising silicon fine particles having a hydrogen-generating ability selected from the group consisting of
(1) crystal grains of silicon having a particle size of 1 µm to 2 µm measured by an X-ray diffractometer;
(2) silicon particles having a particle size of 1 µm or more measured by an X-ray diffractometer and a surface area equivalent to that of silicon fine particles having a crystallite diameter of 1 nm to 100 nm measured by an X-ray diffractometer; and
(3) fine particles of 100 nm² or more that have a surface area equivalent to that of silicon fine particles having a crystallite diameter of 1 nm to 100 nm measured by an X-ray diffractometer; and are (a) porous, (b) polycrystalline, or (c) unfree, hardly crushed or granulated with an undecomposed binding agent.

2. The drug for use according to claim 1, wherein the drug is prepared to be suitable for oral ingestion.

3. The drug for use according to claim 1 or 2, wherein
the silicon fine particle does not pass through a cell membrane of an intestinal tract and between cells of the intestinal tract,
and
the crystal grain does not pass through the cell membrane and between the cells.

4. The drug for use according to any one of claims 1 to 3, further comprising a pH value adjusting agent that sets a pH value of a water-containing liquid to more than 7 and less than 10 when the drug is disintegrated in pure water.

5. The drug for use according to any one of claims 1 to 4, wherein the drug comprises an enteric coating agent containing a bicarbonate.

6. The drug for use according to any one of claims 1 to 5, wherein an administration method is oral administration of an enteric coating agent containing the silicon fine particle, or the crystal grain, and a bicarbonate

7. The drug for use according to any one of claims 1 to 6, wherein the drug has a sustained release property.

8. The drug for use according to any one of claims 1 to 7, wherein the silicon fine particle substantially includes a silicon fine particle having a crystallite diameter of 1 nm or more and 100 nm or less measured by an X-ray diffractometer.

## Patentansprüche

1. Arzneimittel zur Verwendung bei einem Vorbeugen oder Behandeln einer Varikozele als einer Fortpflanzungsstörung bei männlicher Unfruchtbarkeit, das Silicium-Feinpartikel mit einer Fähigkeit zur Wasserstofferzeugung umfasst, die aus der Gruppe ausgewählt sind, die aus
(1) Kristallkörnern von Silicium, die eine Partikelgröße von 1 µm bis 2 µm, gemessen mittels Röntgendiffraktometer, aufweisen,
(2) Siliciumpartikeln, die eine Partikelgröße von 1 µm oder mehr, gemessen mittels Röntgendiffraktometer, und eine Oberfläche, die zu derjenigen von Silicium-Feinpartikeln mit einem Kristallitdurchmesser von 1 nm bis 100 nm, gemessen mittels Röntgendiffraktometer, äquivalent ist, aufweisen,
(3) Feinpartikeln von 100 nm² oder mehr, die eine Oberfläche aufweisen, die zu der von Silicium-Feinpartikeln mit einem Kristallitdurchmesser von 1 nm bis 100 nm, gemessen mittels Röntgendiffraktometer, äquivalent ist, und (a) porös, (b) polykristallin oder (c) unfrei, kaum zerkleinert oder mit einem unzersetzten Bindemittel granuliert sind,
besteht.

2. Arzneimittel zur Verwendung gemäß Anspruch 1, wobei das Arzneimittel so zubereitet ist, dass es für eine orale Einnahme geeignet ist.

3. Arzneimittel zur Verwendung gemäß Anspruch 1 oder Anspruch 2, wobei das Silicium-Feinpartikel nicht durch eine Zellmembran des Intestinaltrakts und zwischen den Zellen des Intestinaltrakts hindurch tritt,
und
das Kristallkorn nicht durch die Zellmembran und zwischen den Zellen hindurch tritt.

4. Arzneimittel zur Verwendung gemäß einem der Ansprüche 1 bis 3, das des Weiteren ein Mittel zur Einstellung des pH-Werts umfasst, das den pH-Wert einer wasserhaltigen Flüssigkeit auf mehr als 7 und weniger als 10 einstellt, wenn das Arzneimittel in reinem Wasser aufgelöst wird.

5. Arzneimittel zur Verwendung gemäß einem der Ansprüche 1 bis 4, wobei das Arzneimittel ein magensaftresistentes Beschichtungsmittel, das ein Bicarbonat beinhaltet, umfasst.

6. Arzneimittel zur Verwendung gemäß einem der Ansprüche 1 bis 5, wobei es sich bei einem Verabreichungsverfahren um eine orale Verabreichung eines magensaftresistenten Beschichtungsmittels, das die Silicium-Feinpartikel oder die Kristallkörner sowie ein Bicarbonat beinhaltet, handelt.

7. Arzneimittel zur Verwendung gemäß einem der Ansprüche 1 bis 6, wobei das Arzneimittel die Eigenschaft einer verzögerten Freisetzung aufweist.

8. Arzneimittel zur Verwendung gemäß einem der Ansprüche 1 bis 7, wobei das Silicium-Feinpartikel im Wesentlichen ein Silicium-Feinpartikel umfasst, das einen Kristallitdurchmesser von 1 nm oder mehr und 100 nm oder weniger, gemessen mittels Röntgendiffraktometer, aufweist.

## Revendications

1. Médicament destiné à être utilisé dans la prévention ou le traitement de la varicolèle en tant que trouble reproductif de l'infertilité masculine comprenant de fines particules de silicium ayant une capacité à produire de l'hydrogène choisies dans le groupe constitué par
(1) des grains de cristal de silicium ayant une taille de particule de 1 µm à 2 µm mesurée par un diffractomètre aux rayons X ;
(2) des particules de silicium ayant une taille de particule de 1 µm ou plus mesurée par un diffractomètre aux rayons X et une surface équivalente à celle des fines particules de silicium ayant un diamètre de cristallite de 1 nm à 100 nm mesuré par un diffractomètre aux rayons X ; et
(3) des fines particules de 100 nm² ou plus qui ont une surface équivalente à celle de fines particules de silicium ayant un diamètre de cristallite de 1 nm à 100 nm mesuré par un diffractomètre aux rayons X et sont (a) poreuses, (b) polycristallines ou (c) non libres, à peine écrasés ou granulées avec un agent de liaison non décomposé.

2. Médicament destiné à être utilisé selon la revendication 1, dans lequel le médicament est préparé pour être approprié pour l'ingestion orale.

3. Médicament destiné à être utilisé selon la revendication 1 ou 2, dans lequel
la fine particule de silicium ne passe pas à travers une membrane cellulaire d'un tractus intestinal et entre les cellules du tractus intestinal,
et
le grain de cristal ne passe pas à travers la membrane cellulaire et entre les cellules.

4. Médicament destiné à être utilisé selon l'une quelconque des revendications 1 à 3, comprenant en outre un agent d'ajustement de valeur de pH qui règle une valeur de pH d'un liquide contenant de l'eau à plus de 7 et moins de 10 quand le médicament est désintégré dans l'eau pure.

5. Médicament destiné à être utilisé selon l'une quelconque des revendications 1 à 4, dans lequel le médicament comprend un agent de revêtement entérique contenant un bicarbonate.

6. Médicament destiné à être utilisé selon l'une quelconque des revendications 1 à 5, dans lequel un procédé d'administration est l'administration orale d'un agent de revêtement entérique contenant la fine particule de silicium ou le grain de cristal et un bicarbonate.

7. Médicament destiné à être utilisé selon l'une quelconque des revendications 1 à 6, dans lequel le médicament a une propriété de libération prolongée.

8. Médicament destiné à être utilisé selon l'une quelconque des revendications 1 à 7, dans lequel la particule fine de silicium comprend essentiellement une fine particule de silicium ayant un diamètre de cristallite de 1 nm ou plus et de 100 nm ou moins mesuré par un diffractomètre aux rayons X.
